# EUROPEAN PATENT APPLICATION

(11) **EP 2 072 042 A1**
(43) Date of publication of application: **24.06.2009**
(21) Application number: 07150359.3
(22) Date of filing: 21.12.2007
(51) Int. Cl.: A61K 9/18, A61K 31/122, A61K 31/397

(54) **Active pharmaceutical ingredient on solid support, amorphous and with improved solubility**

(71) Applicant: Lek Pharmaceuticals D.D., 1526 Ljubljana (SI)
(72) Inventor: Grahek, Rok, 4000, Kranj (SI); Resman, Aleksander, 1000, Ljubljana (SI); Batarda, Andrej, 1360, Vrhnika (SI); Crnugelj, Martin, 1000, Ljubljana (SI)
(74) Representative: Kröger, Bernd

(57) **Abstract**

The present invention discloses a combination preparation comprising an active pharmaceutical ingredient (API) and a pharmaceutically acceptable solid support,
wherein said solid support is in a water-insoluble particulate form and comprises a material selected from silicic acid, aluminum hydroxide and titanium hydroxide, wherein said API is a compound having both, at least one hydrophobic structural moiety causing the API to have low solubility in water or in an aqueous solution, and multiple hydrophilic groups arranged to form multiple intermolecular interactions to said solid support involving polar or ionic or hydrogen bonding, wherein the hydrophilic groups of the API are independently equal or different and comprise at least one of the group consisting of OH- and halogen-groups;
wherein said API is bound to said solid support by adsorption, and wherein a major proportion of said API is in the amorphous state.

The present invention also discloses a pharmaceutical preparation comprising such a combination preparation as well as a method for producing such a combination preparation.

## Description

The present invention relates to a combination of poorly soluble active pharmaceutical ingredients (APIs) with pharmaceutically acceptable solid supports. The present invention further relates to a pharmaceutical preparation comprising such a combination preparation as well as to a method for producing such a combination preparation.

An active pharmaceutical ingredient (API), i.e. a substance or a compound that is intended to be used for a pharmaceutical product as a therapeutically active compound (ingredient), sometimes involves problems of low solubility in water or aqueous solutions or in use environments. In the past, various attempts have been made to improve solubility of APIs.

EP 129 893 A, for example, proposes to increase solubility by preparing a ground mixture of a poorly soluble crystalline drug with an adsorbant. As adsorbants, solid supports such as activated charcoal, activated clay, silica, synthetic adsorbant resin, activated alumina and other physiologically acceptable adsorbants are mentioned.

WO 03/000238 A in the introduction refers to prior attempts to improve solubility by the formation of adsorbates, and it then teaches about an increased solubility of a low-solubility API when the active drug is adsorbed on a solid support mainly in amorphous form by rapid evaporation of an organic solvent, such as spray drying, from a suspension containing a corresponding low-solubility drug and the solid substrate. The API preferably is a CCR1 inhibitor or a CETP inhibitor, and a wide range of inorganic and polymer supports are contemplated. The document further teaches that the surface of the substrate may be modified with various substituents, such as varying the terminal groups of substituents attached to the substrate in order to influence the interaction between the drug and the substrate. Where the drug is hydrophobic, the document indicates that it may be desired to select a substrate having hydrophobic substituents to improve the binding of the drug to the substrate. A concentration-enhancing polymer is preferably co-adsorbed with the drug to the substrate.

The object of the present invention was to improve the performance of combinations between APIs and pharmaceutically acceptable solid supports. In particular, an improved solubility in water or aqueous solutions compared to the solubility of the free form of the same API shall be achieved and applicable to various possible APIs in spite of structurally different configurations, while improving the system economy.

The object is solved by the provision of a combination preparation as defined in claim 1. Preferred embodiments are set forth in the sub-claims thereto. The present invention further provides a pharmaceutical preparation comprising the combination preparation of the present invention as well as a method for producing such a combination preparation according to claims 15 and 16, respectively. A preferred production method is set forth in sub-claim 17.

According to the present invention, it was surprisingly found that not only the type and the nature of the solid support, but especially the combination thereof with an appropriate proportion of hydrophobic structural moieties and hydrophilic groups existing in the structure of the API are important for not only ensuring the adsorbed API to be mainly in the amorphous state, but particularly for providing specific and concerted interaction between the API and the solid support which appears to act synergistically for achieving a proper binding (in organic solvents), yet a relatively fast and effective dissociation (in water, aqueous solvents or use environments) therebetween. As the API's hydrophilic groups are defined by at least one, preferably two or more of OH- and halogen- (Hal) groups, and in particular at least one, and more preferably two or more groups of each of OH and Hal (especially F and/or Cl), multiple strong hydrogen bonding and polar or ionic group interactions are formed during the adsorption process (because of which a slow evaporation adsorption process is more preferable than fast evaporation), which subsequently, when placed in an aqueous environment, allows multi-site interaction points for solubilizing water molecules and hence significantly enhanced solubility, in spite of the presence of hydrophobic structural moieties which would cause the API as such, in a comparative free form, to have low solubility in water or in an aqueous solution.

Moreover, apparently due to the presence of multiple sites of polar or ionic intermolecular interaction or hydrogen bonding between the appropriate hydrophilic groups of the API and the specific solid support having surface OH-groups, regular interaction between the APIs can be disturbed and thus crystallization of the API is inhibited, thereby ensuring an enhanced amorphous proportion of the adsorbed API which in turn further contributes to a higher solubility for a given API. Further surprisingly, this can be achieved even with normally evaporating organic solvent during the adsorption process, thereby obviating special process requirements such as fast solvent evaporation by spray drying, spray coating, rapid mixing precipitation or thermal melt-extrusion processes. This particularly contributes the to system economy.

According to a preferred embodiment of the combination preparation of the present invention, it is possible to achieve an interaction between the API and the solid support which can be characterized by differential scanning calorimetry (DSC) spectrum such that any transition characteristic of the corresponding free form of a given API alone is not indicated. High proportions of amorphousness is ensured, reaching levels of at least 75%, preferably at least 90% and more preferably at least 95% and even reaching 100 % of the adsorbed API being in the amorphous state. It has been found that, although being otherwise highly hydrophobic and thus showing low solubility in water or aqueous solutions, the presence of multiple OH- and halogen-groups, in particular when 1, 2, 3 or more of each of OH- and Hal-(F or Cl) substituents are combined, essentially the whole amount or even the complete amount of the API adsorbed on the specific solid support is amorphous and subsequently shows markedly improved solubility. In this respect, dual-type and multi-type interactions between the selected API and the hydrophilic groups of the specific solid support are considered to be advantageous.

The combination preparation according to the present invention provides beneficial features by displaying a solubility of the selected API in water or an aqueous solution being increased by a factor of at least 1.5, preferably at least 2.0 and even at least 3.0 relative to a referenced free form of the same API, which is unbound and either amorphous or crystalline, in the same aqueous solution. The aforementioned marked solubility increase is more preferably valid also relatively to the form of the physical mixture of the same API and the same solid support, i.e. a comparison without an adsorption treatment.

Hence, the present invention provides a beneficial and reliable platform technology which, when observing the structural requirements of both the specific solid support and the selected API as defined in claim 1 and as more preferably defined in the sub-claims, displays an improved performance by realizing high proportions of amorphous APIs adsorbed on the solid support, enhanced solubility in water and aqueous solutions as well as use environments, combined with the system economy and freedom to select appropriate API molecules within reasonable limits.

In the following, the present invention, its concept and principle as well as preferred embodiments and illustrative examples will be explained in more detail, while noting that the specific embodiments and examples are for illustrative purposes only and do not intend to limit the invention. In the drawings:
Fig. 1 shows dissolution profiles of an API adsorbed on a silicic acid support (ezetimibe at a mass fraction of 15 %), compared with a comparison sample of a physical mixture (i.e. no adsorbed form) of the same amount and type of API and the same silicic acid support, and compared with the amorphous free API and the crystalline free API, respectively in water.
Fig. 2 shows a dissolution profile of an API (ezetimibe at a mass fraction of 5 %) adsorbed on silicified microcrystalline cellulose containing 2 wt. % colloidal fumed silica and 98 wt. % microcrystalline cellulose, compared with the crystalline free form of the same API, respectively in water.
Fig. 3 shows a dissolution profile of an API adsorbed on a silicic acid solid support (atovaquone at a mass fraction of 5 %) compared with the crystalline free form of the same API, respectively in 10 mM triethylamine / HCl (pH 10) buffer.

The present platform is applicable to APIs selected to have at least one hydrophobic structural moiety, due to which the API *per se* has a low solubility in water or in an aqueous solution and therefore solubility-enhancement becomes relevant. For example, hydrophobic structural moieties causing the API to have a low solubility usually comprise alkyl, alkylene, alkyne, respectively either straight chains or branched chains, cyclic and/or aromatic forms thereof, including phenyl, naphthyl, which moieties optionally are substituted or contain one or more heteroatoms such as O, N, S or the like.

Due to the presence of at least one hydrophobic structural moiety, the concept of the present invention is beneficially applicable to APIs having a low solubility in water or in an aqueous solution. For example, low soluble APIs usefully applied in the present invention have solubility in water (at 20 °C at around pH 7-8) of about 10 mg/ml or lower, preferably about 1 mg/ml or lower, more preferably about 100 µg/ml or lower, and particularly about 10 µg/ml or lower. As another indication for the hydrophobicity of the APIs useful for the present invention, their log P values (octanol-water) typically are in the range of about 3 or higher, preferably about 4 or higher and even about 6 or higher.

In addition, the selected API contains multiple, i.e. at least 2, preferably 3, 4, 5, 6, 7, 8 or more hydrophilic groups. Among these hydrophilic groups bound to the selected API, preferably at least 2, more preferably at least 3 and particularly 4 or more hydrophilic groups are selected from OH- and halogen-groups. More preferably, the API has at least one, preferably 2 or more OH-groups, and at least one, preferably 2 or more of F and Cl. The aforementioned hydrophilic groups are preferably distributed over the whole structure of the API, i.e. are bonded at different positions and sections of the whole compound. The API preferably has further hydrophilic groups susceptible of forming polar, ionic, dipole-dipole or hydrogen bond interactions to the hydrophilic OH-groups of the specific solid support, which groups are different from the aforementioned OH- and Halogen-groups. These other groups preferably include, but are not limited to amine-, amid-, carboxylic acid-, ester-, chinoline-, beta-diketone-, carbonyl-, sulfonyl-, sulfonamino-, sulfonamido- and sulfonate-groups. These other groups themselves are preferably located at different positions of the API.

The distribution of interaction points between the selected API and the specific solid support is another relevant factor. Support binding and dissociation of low soluble APIs is appropriately balanced, when the structural configuration within the API has at least 3 equal or different hydrophobic structural units, to each of which at least one of the hydrophilic groups as defined above and preferably at least one of OH- or halogen-groups are respectively bonded.

According to the present invention, substantial amounts of the selected API can be adhered to the specific solid support, while still providing improved solubility. As suitable adsorption ratio is up to 50 wt. %, preferably up to 20 wt. %, more preferably up to 15 wt. %, and particularly up to 10 wt. %. As a lower adsorption ratio limit, at least 0.05 wt. %, preferably at least 1 wt. % and particularly at least 2 wt. % are suitable.

It has been shown that particular effects according to the present invention are accomplished in combination when using, as representative APIs, ezetimibe (1-(4-fluorophenyl)-3-[3-(4-fluorophenyl)-3-hydroxy-propyl]-4-(4-hydroxyphenyl)-azetidin-2-one) and atovaquone (3-[4-(4-chlorophenyl)cyclohexyl]- 4-hydroxy-naphthalene-1,2-dione) shown in the following formulae:

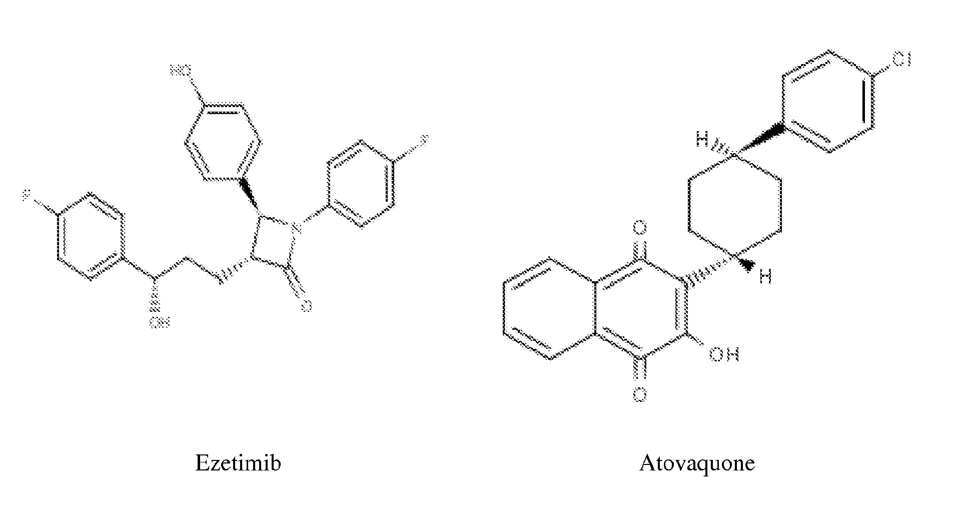

In spite of basically different actual structures of the APIs tested, the common features displaying both hydrophobic and hydrophilic properties confirm the concept of the present invention and its preferred embodiments as explained above. Owing to the particular configuration and distribution of the opposite structural features, i.e. hydrophobic structural units having appropriate hydrophilic groups to form multiple interaction sites with the specific solid support, similar effects are achievable by other corresponding APIs according to the concept of the present invention.

Accordingly, the platform technology of the present invention is, for example, applicable to APIs such as thyroxine, benziodarone, amiodarone, enoxacin, 3-Hydroxy-2-(1,3-indandione-2-yl)quinoline, esketamin, etacrynic acid, chlorophenothane, fenoterol, 1,8,9-anthratriol, cyproterone, flutamide, clenbuterol, bromocryptine, benperidol, statins such as fluvastatin, pravastatin and lovastatin, carvedilol, mefloquine, ramipril, furosemid, piretanide, pentazocine, miconazole, tricyclohexyltin hydroxide, monensin, prednimustine, misoprostol, clopimozide, brodifacoum, itraconazole, metipranolol, hydroxychloroquin, fluticasone, etc., without however being limited thereto.

As a further relevant requirement for forming multiple interaction sites involving hydrogen bonding, the solid support comprises a material selected from silicic acid, aluminum hydroxide and titanium hydroxide. This material component of the solid support can be defined by structural formulae Si(OH)₄, SiO₂ x nH₂O, Al(OH)₃, Al₂O₃ x nH₂O, Ti(OH)₄ or TiO₂ x nH₂O (n respectively being an integer) as well as their anhydrous forms still having hydrophilic groups. The hydrophilic groups are preferably present at surfaces at the outer or at inner pores of the support particles. Particularly preferred are silicic acids and above all its light anhydrous forms, preferably fumed silica, silica shell (silicic acid particle in hollow form), polysilicic acid (SiO₂ x nH₂O), silica gels, further including orthosilicic acid, metasilicic acid, mesodisilicic acid, mesotrisilicic acid, mesotetrasilicic acid, etc. Further contemplated are mixtures or core/shell structures or core/coating-structures involving at least one of the afore-mentioned materials selected from silicic acid, aluminum hydroxide and titanium hydroxide as a component thereof.

The material selected from silicic acid, aluminum hydroxide and titanium hydroxide as described above is preferably in a fine particulate form, suitably having a mean (primary) particle size of at least 5 nm, more preferably at least 10 nm, and suitably ranging up to 500 µm, preferably 200 µm and more preferably 100 µm. A preferred (primary) particle size ranges from 7 nm to 1 µm, more preferably from 10 nm to 100 nm. The primary particles may form aggregates, or they may be combined with other water-insoluble support materials. The material selected from silicic acid, aluminum hydroxide and titanium hydroxide in a fine particulate form preferably has a specific BET surface area of at least 10 m²/g, preferably at least 50 m²/g and more preferably at least 150 m²/g.

In accordance with a preferred embodiment, the material selected from silicic acid, aluminum hydroxide and titanium hydroxide in a fine particulate form is mixed with, and preferably adhered to a further solid, hydrophilic and particulate support matrix. This additional support matrix may serve for additional function as a pharmaceutical excipient, in particular for improving flowability and possibly other excipient functions. A particularly preferred additional solid support matrix is microcrystalline cellulose. An example for providing a whole solid support beneficially usable as a solid support for the present invention is silicified microcrystalline cellulose comprising colloidal fumed silica and microcrystalline cellulose.

In order to effectively display the effects of the present invention, the material selected from silicic acid, aluminum hydroxide and titanium hydroxide is hydrophilic and therefore should not be modified by a hydrophobization treatment, and more preferably no substance other than the API is co-adsorbed onto the specific solid support to be used for the combination preparation according to the present invention. This also improves the system economy.

As a preferred and beneficial method for producing the combination preparation according to the present invention described above, steps of providing a suspended solution comprising the specific solid support, the selected API and an organic solvent dissolving the API, then removing the organic solvent by evaporation, and subsequently obtaining a combination preparation having the API bound to the solid support by adsorption, wherein a major proportion of the API is in the amorphous state. For providing the suspended solution, the API is preferably first dissolved in the organic solvent, and the solid support is added thereto subsequently. Examples of suitable solvents include, but are not limited to dialkyl ketone such as t-butyl-methyl-ketone, hexane, diethyl ether, tetrahydrofuran, toluene, ethylacetate, acetonitril, ethanol, tetrachloroethylene, chloroform, and mixtures thereof. The suspension is preferably agitated during the adsorption step. The organic solvent is then removed by evaporation at normal atmosphere or at partial vacuum. Evaporation is preferably carried out such that the organic solvent is evaporated slowly, suitably during a period of at least 30 minutes, more preferably at least 2 hours. It is assumed that slow evaporation contributes to a well-oriented interaction between the specific solid support and the particular API, enabling the adsorption process to be more effective and thorough. Furthermore, it is assumed that both the structural requirements and the slow evaporation process promote monolayer formation and thus establish a favorable situation for the subsequent solubilization step in water, an aqueous solution or a use environment. In addition, the method for producing the combination preparation according to the present invention beneficially avoids uneconomic, complex and laborious process steps and equipments, which would otherwise be necessary for fast evaporation, ground mixing or thermal extrusion processes.

The combination preparation according to the present invention is beneficially used as a pharmaceutical preparation. A disease usually treated by the particular selected API can be effectively treated, with the associated advantage that the specifically adsorbed form on the solid support according to the present invention provides a markedly improved solubility. As a consequence, bioavailability of the selected API can be improved as well.

Alternatively, besides using the combination preparation obtained according to the present invention as such, the active API may instead be dissociated from the solid support and subsequently used for a medical treatment in a free form solubilized in an aqueous solution. Owing to the markedly increased solubility, the combination with the specific solid support serves as a beneficial vehicle for medical applications.

The pharmaceutical preparation comprising the combination preparation described above may contain further pharmaceutically acceptable excipients.

The pharmaceutical preparation may comprise, besides the combination preparation described above, a further active drug different from the API adsorbed on the solid support.

When used, the pharmaceutically acceptable excipients may be selected from those generally used in the connection with the respectively selected API. This may also depend on the respectively appropriate administration, in particular including but not limited to oral administration or any other suitable administration forms. A correspondingly suitable formulation and dosage form may be chosen as well, including but not limited to tablet, pill, capsule, suspension, emulsion, oil, another solid, semi-solid, gelatinous or hydrogel-like, or liquid form, a powder, an ointment, a cream, an oil, a modified release dosage form, etc. The pharmaceutical preparation containing the combination preparation according to the present invention is preferably formulated as an oral administration form. In accordance with the preferred embodiment, the support material used for adsorption of the selected API not only contains the binding support selected from silicic acid, aluminum hydroxide and titanium hydroxide, but additionally performs further excipient function such as flowability. For this embodiment, silicic acid, aluminum hydroxide and/or titanium hydroxide is most preferably combined with microcrystalline cellulose to form a usable solid support, and a further excipient for the whole pharmaceutical preparation can be omitted, if desired.

The combination preparation of the present invention furthermore provides an appropriate storage form for the respective API. Furthermore, the relatively mild conditions, which can applied according to the process of the present invention during the adsorption process, are in favor of stability of a given API.

### Examples

### Example 1:

0.15 g of ezetimibe (logP/hydrophobicity value of 4.584) was dissolved in 50 ml of a mixture of 20 % of hexane in tert-butyl methyl ketone and 0.85 g of fumed silica (Aerosil^{™} 200) was added. Mass fraction of ezetimibe was 15 %. Subsequently the solvent was removed by evaporation.

### Example 2:

0.05 g of ezetimibe was dissolved in 20 ml of a mixture of 20 % of hexane in tert-butyl methyl ketone and 0.95 g of colloidal silicon dioxide (CSD; 2wt. %) on microcrystalline cellulose (MCC; 98 %) (Prosolv^{™}) was added. Mass fraction of ezetimibe was 5 %. Subsequently the solvent was removed by evaporation.

### Example 3:

0.05 g of atovaquone (logP/hydrophobicity value of 5.251) was dissolved in 20 ml of a mixture of 10 % of hexane in tert-butyl methyl ketone and 0.95 g of fumed silica (Aerosil^{™} 200) was added (mass fraction of atovaquone was 5 %). Subsequently the solvent was removed by evaporation.

### Example 4:

Samples were analysed by using differential scanning calorimetry (DSC), dynamic vapour sorption (DVS) and X-ray powder diffraction (XRPD).

Results from DSC and DVS showed that interaction between the API and the solid support exists. DSC spectra showed no transitions characteristic of the API. In addition, hygroscopicity is lower compared to a physical mixture of the API and the solid support.

Furthermore reduction in specific surface area of the solid support due to the presence of the API was observed. These results confirm that adsorption of the API on the solid support takes place.

Using XRPD it was confirmed that in the case of ezetimibe on Aerosil and Prosolv (examples 1 and 2), the API was amorphous while in the case of atovaquone on aerosil (example 3) small amounts of crystalline API were detected.

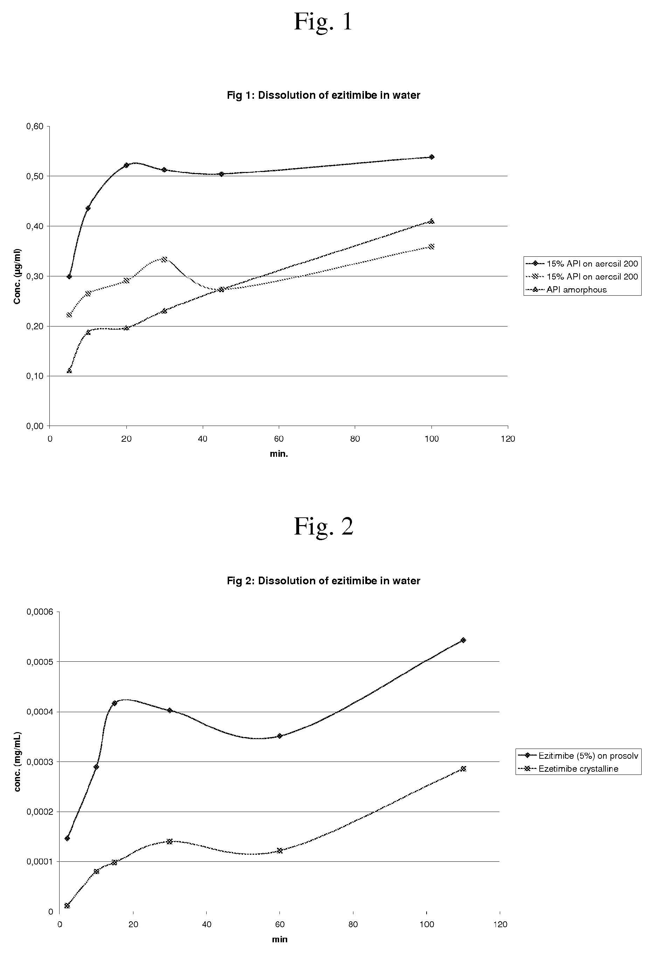

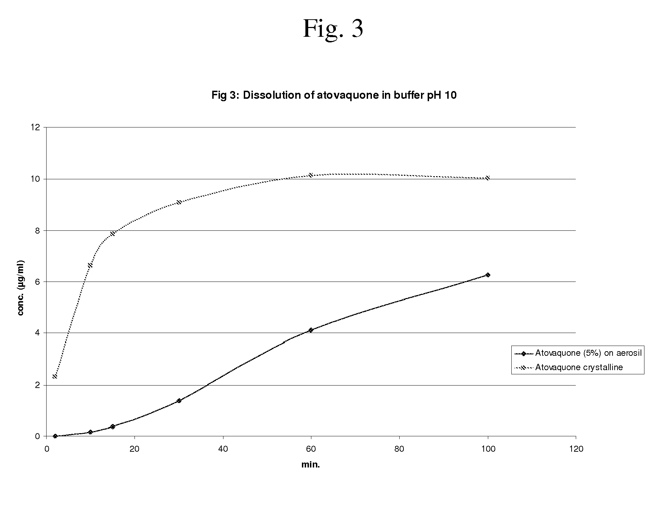

In all three examples, the API on the solid support showed markedly improved solubility. The results are shown in Tables 1 to 3, and Figures 1 to 3.

**Table 1. Ezetimibe and Aerosil 200**

| | Amount dissolved (µg/ml) | | |
|---|---|---|---|
| Time (min.) | API (15 %) on solid support | Physical mixture | Ezetimibe amorphous |
| 10 | 0.44 | 0.26 | 0.19 |
| 20 | 0.52 | 0.29 | 0.20 |
| 45 | 0.5 | 0.27 | 0.27 |
| 100 | 0.54 | 0.36 | 0.41 |

**Table 2. Ezetimibe and Prosolv**

| | Amount dissolved (µg/ml) | |
|---|---|---|
| Time (min.) | API (5 %) on solid support | Crystalline ezetimibe |
| 10 | 0.29 | 0.08 |
| 20 | 0.42 | 0.13 |
| 60 | 0.35 | 0.12 |
| 100 | 0.5 | 0.25 |

**Table 3. Atovaquone and Aerosil 200**

| | Amount dissolved (µg/ml) | |
|---|---|---|
| Time (min.) | API (5 %) on solid support | Crystalline atovaquone |
| 10 | 6.6 | 0.16 |
| 20 | 8.5 | 0.7 |
| 60 | 10.1 | 4.1 |
| 100 | 10 | 6.2 |

## Claims

1. A combination preparation comprising an active pharmaceutical ingredient (API) and a pharmaceutically acceptable solid support,
wherein said solid support is in a water-insoluble particulate form and comprises a material selected from silicic acid, aluminum hydroxide and titanium hydroxide,
wherein said API is a compound having both, at least one hydrophobic structural moiety causing the API to have low solubility in water or an aqueous solution, and multiple hydrophilic groups arranged to form multiple intermolecular interactions to said solid support involving polar or ionic or hydrogen bonding, wherein the hydrophilic groups of the API are independently equal or different and comprise at least one of the group consisting of OH- and halogen-groups;
wherein said API is bound to said solid support by adsorption, and wherein a major proportion of said API is in the amorphous state.

2. The combination preparation according to claim 1, wherein the combination of said solid support and said API being adsorbed thereon displays a solubility in water or in an aqueous solution being increased by a factor of at least 1.5 relative to a referenced free form of the same API in the same aqueous solution.

3. The combination preparation according to claim 1 or 2, which is **characterized by** an interaction between said API and said solid support such that a differential scanning calorimetry (DSC) thermogram of the combination preparation does not show any transitions characteristic of the corresponding free form of said API alone.

4. The combination preparation according to any one of the preceding claims, wherein said API has at least 2, preferably at least 3 or 4 or more hydrophilic groups selected from OH- and halogen-groups.

5. The combination preparation according to any one of the preceding claims, wherein said API has both at least 1, preferably at least 2 OH-groups and at least 1, preferably at least 2 halogen-groups.

6. The combination preparation according to any one of the preceding claims, wherein said API has further hydrophilic groups other than OH- and halogen-groups, the other groups being selected from chinoline-, beta-diketone-, amino-, amid-, carboxylic acid- and ester-groups.

7. The combination preparation according to any one of the preceding claims, wherein said API is **characterized by** the following structural configuration:
● presence of at least 3 hydrophobic moieties, independently being same or different;
● each of at least 2 of said hydrophobic moieties are respectively bonded to at least one of said hydrophilic groups.

8. The combination preparation according to any one of the preceding claims, wherein said API is ezetimibe.

9. The combination preparation according to any one of claims 1 to 7, wherein said API is atovaquone.

10. The combination preparation according to any one of the preceding claims, wherein the said solid support material comprises silicic acid.

11. The combination preparation according to any one of the preceding claims, wherein the said solid support material comprises fumed silica or colloidal silica.

12. The combination preparation according to any one of the preceding claims, wherein said solid support is silicified microcrystalline cellulose.

13. The combination preparation according to any one of the preceding claims, wherein no substance other than said API is co-adsorbed onto said solid support.

14. The combination preparation according to any one of the preceding claims, which is obtainable by a process as defined by claim 16 or 17.

15. A pharmaceutical preparation comprising a combination preparation as defined in any one of the preceding claims.

16. A method for producing a combination preparation comprising an active pharmaceutical ingredient (API) and a pharmaceutically acceptable solid support, the method comprising:
a) providing a suspended solution comprising:
● a solid support which is in a hydrophilic particulate form and comprises a material selected from silicic acid, aluminum hydroxide and titanium hydroxide;
● an API having both, at least one hydrophobic structural moiety causing the API to have low solubility in water or in an aqueous solution, and multiple hydrophilic groups arranged to form multiple intermolecular interactions to said solid support involving polar or ionic or hydrogen bonding, wherein the hydrophilic groups of the API are independently equal or different and comprise at least one of the group consisting of OH- and halogen-groups; and
● an organic solvent dissolving said API
b) removing said organic solvent by evaporation; and
c) obtaining a combination preparation having said API bound to said solid support by adsorption, wherein a major proportion of said API is in the amorphous state.

17. The process according to claim 16, wherein said step b) is carried out by a slow evaporation of said organic solvent during a period of more than about 30 min.
